# EUROPEAN PATENT SPECIFICATION

(11) **EP 1 444 952 B1**
(45) Date of publication and mention of the grant of the patent: **07.05.2008**
(21) Application number: 03405069.0
(22) Date of filing: 10.02.2003
(51) Int. Cl.: A61B 6/06

(54) **Scanning digital radiography system with reduced ionizing-radiation dose**
Abtastendes digitales Radiographie-System mit verminderter ionisierender Strahlendosis
Système numérique de radiographie à balayage avec dose réduite de rayonnement ionisant

(43) Date of publication of application: 11.08.2004
(73) Proprietor: CSEM Centre Suisse d'Electronique et de Microtechnique SA, 2007 Neuchâtel (CH)
(72) Inventor: Hinderling, Thomas, CH-2000 Neuchâtel (CH); Schmidiger, Thomas, CH-6072 Sachseln (CH); Busse-Grawitz, Max Erick, CH-6064 Kerns (CH)
(74) Representative: Schneider Feldmann AG Patent- und Markenanwälte

(56) References cited:
- EP-A- 1 216 661
- WO-A-01/35829
- US-A- 6 055 295
- US-A1- 2001 031 032
- US-B1- 6 501 828
- MIYAZAKI OSAMU ET AL: "New CT scanning approach for decreasing radiation dose" MEDICAL IMAGING 1998: IMAGE DISPLAY;SAN DIEGO, CA, UNITED STATES FEB 22-24 1998, vol. 3335, 1998, pages 655-662, XP008019632 Proc SPIE Int Soc Opt Eng;Proceedings of SPIE - The International Society for Optical Engineering 1998

## Description

### Field of the invention

This invention relates generally to radiography, and specifically to a scanning digital radiography method and apparatus according to the preamble of the independent claims. Its possible applications are, for instance, in scanning digital radiography exposing patients to ionizing radiation or in material inspection with reduced ionizing-radiation dose.

### Background of the invention

Contemporary scanning digital radiography systems consist of an X-ray source with statically programmable excitation voltage and current, a linear array of X-ray sensitive detector elements with digital output, a source collimator whose geometry can be manually adapted, a transport mechanism for the linear detector array that is synchronized with the source collimator, and a control unit. However, the disadvantages of prior-art systems are: (1) no provision is made for selecting a concise area of interest; (2) the area of interest is limited to rectangular geometries; and (3) the X-ray radiation parameters, intensity and energy, are not optimized to keep the signal-to-noise ratio within predefined, object-determined limits.

U.S. Patent No. 5,054,048 describes a method and a device for producing flux-equalized X-ray images for medical radiography that makes use of a scanning fan-shaped X-ray beam and a feedback control system, which regulates the beam intensity at a multiple number of points along the fan beam to compensate for the X-ray attenuation variations of the patient. Its teaching allows beam equalization in the direction of the scan and in the direction perpendicular to the scan. The objective of this two-dimensional beam equalization is to adjust the X-ray quanta to the narrow exposure range of conventional high contrast enhancement film-screen systems. The disadvantages of the teaching of this reference are: (1) additional X-ray sensors are needed for control feedback; (2) the probability of artifacts caused by the control feedback is large since the calculation of the X-ray-source parameters, X-ray intensity and energy, is based only on the current feedback without knowledge of the structure of the complete region of interest; (3) the region of interest is limited to rectangular shapes; and (4) since the film-screen systems have an extremely narrow exposure range, the controller tries to equalize signal intensity resulting in sub-optimal signal-to-noise ratios and radiation dose.

U.S. Patent No. 6,256,406 describes a system for acquiring diagnostic X-ray images having uniform optical density. Such images are generated by selectively scanning lines of pixels from a matrix detector according to exposure compensation profiles. The exposure compensation profiles include an ordered sequence of exposure times that can be loaded into a programmable timer coupled to the readout circuitry of the matrix detector. The exposure compensation profiles can be generated from empirical data by taking preliminary dose exposures of the body part to be examined. However, the disadvantages of the teaching of this reference are: (1) the X-ray dose exposed to the patient is high since the X-ray system exposes all lines of pixels with the highest necessary dose; (2) the X-ray-source parameters, intensity and energy, are not optimized for each line; and (3) the low dose exposure is only used to calculate the exposure compensation profiles but is not used to select a region of interest of any shape and to derive the optimal X-ray-source settings for every line.

U.S. Patent No. 6,243,440 describes an apparatus using a two-dimensional radiation sensor for acquiring X-ray penetration images. A radiographic condition for the production radiography (image acquisition), especially exposure time and X-ray-tube current, is computed based on the ratio between a quantity of charges in a region of interest collected from the two-dimensional radiation sensor during the low dose monitoring radiography and a desired quantity of charges in the region of interest during the production radiography. The production radiography is carried out based on the computation to acquire a high-quality image. Further, it also eliminates the need of separate phototimers in order to avoid underexposure and overexposure. However, the disadvantage of the teaching of this reference is that the X-ray-source parameters, intensity and energy, are optimized for all lines of pixels simultaneously and not for each single line separately resulting in locally sub-optimal X-ray-source parameters.

U.S. 2001/0031032 discloses a computed tomography method for forming a scannogram of adequate image quality, this method requiring a first dose which is not larger than that used for a conventional formation of a scannogram. In this first step, this scannogram enables to define a region of interest to be imaged in a second step by means of a conventional Computed Tomography method. The higher intensity and/or the quality of the radiation to be produced by the radiation source in this second step is calculated in advance.

### Summary of the invention

It is an object of the invention to provide a scanning digital radiography method and apparatus by means of which high-quality images can be acquired with a radiation dose as low as possible. This and other objects are achieved by the method and the apparatus defined in the independent claims.

A preview image is acquired at extremely low radiation and with increased scanning speed. The dose used for the preview-image acquisition is typically in the range of 0.5-10 %, and preferably 2-4 % of the dose necessary for the acquisition of a sound image. The preview image is displayed on a graphical user interface at the operator. The operator highlights the area of interest. The geometry data of the area of interest is transferred to the positioning control unit.

The remotely adaptable collimator of the scanning digital radiography apparatus forms the geometry of the ionizing radiation beam. While the collimator slit width defines the radiation beam's dimension in the direction of motion, the collimator slit length defines how many pixels of the line camera are exposed to ionizing radiation.

After the operator triggers the image acquisition, the positioning control unit moves the detector from the home position to the start point of the area of interest without ionizing radiation. Then, the ionizing radiation is switched on and the area of interest is scanned. Therefore, the positioning control unit synchronously sweeps both, detector and radiation beam over the area of interest, and adapts the collimator slit length continuously to the geometry of the area of interest. Thus, only the area of interest is exposed to ionizing radiation. The architecture of the collimator allows to scan symmetrical and asymmetrical geometries with respect to the collimator centerline. Upon arrival at the end point of the area of interest, the radiation is switched off and the detector is moved back to the home position.

The properties of the ionizing radiation beam are influenced by the excitation voltage and by the current of the ionizing radiation source. While the excitation voltage influences the energy spectrum of the beam, the source current influences the intensity of the beam. In the present invention, a high-voltage generator is used which allows to dynamically modulate the radiation beam's energy spectrum and/or intensity by controlling the ionizing radiation source's excitation voltage and/or excitation current.

The energy spectrum and intensity of the ionizing radiation beam as well as the changing thickness and material of the object of interest influences the signal-to-noise ratio of the image. Using the data from the preview scan, a control unit calculates the local signal-to-noise ratios, compares those calculated signal-to-noise ratios with the predefined object-determined limits, and derives values for the excitation voltage and the tube current for each line. During the image acquisition, the control unit dynamically modulates the excitation voltage and the tube current of the ionizing radiation source according to the pre-calculated values as well as to the already acquired lines of the ongoing acquisition. The result are sound images without additional artifacts and with locally optimized signal-to-noise ratios, acquired at low dose of ionizing radiation.

More particularly, the method for imaging an object with penetrating radiation according to the invention comprises the steps of generating a beam of penetrating radiation, impinging said beam on the object and detecting radiation transmitted through the object, and scanning the object with said beam. A first, preview image is acquired by scanning the object or a part of it with a first radiation dose. An area of interest comprised in said preview image is selected. Thereafter, a second image is acquired by scanning said area of interest with a second radiation dose which is higher than said first radiation dose.

The apparatus for imaging an object with penetrating radiation according to the invention comprises a source for generating penetrating radiation, beam-forming means for forming a beam from said radiation, scanning means for scanning the object with said beam, detector means for detecting radiation generated by said source and transmitted through the object, and control means for controlling the imaging process. Said beam-forming means are suited for dynamically varying the geometry of said beam during a scanning process, being controlled by said control means.

The control means are preferably adapted for making said scanning means scan the object or a part of it with a first radiation dose so as to acquire a first, preview image, selecting an area of interest comprised in said preview image, and making said scanning means scan said area of interest with a second radiation dose which is higher than said first radiation dose so as to acquire a second image.

The beam-forming means for forming a beam from radiation, usable in the apparatus according to the invention, comprise a first pair of shields defining a first slit, and a second pair of shields defining a second slit arranged essentially perpendicular to said first slit. Both pairs of shields are essentially parallel, and both shields of said second pair of shields are movable independently from each other so as to vary the position and/or the width of said second slit.

The present invention improves the prior art regarding the following aspects:
- The geometry of the area of interest is not limited to rectangular shapes eliminating the exposure of ionizing radiation beyond the area of interest. The concise area of interest is selected from a preview acquired with extremely low radiation dose.
- The signal-to-noise ratio is optimized during the acquisition process in order to acquire sound digital images with minimized radiation.
- The X-ray-source parameters, intensity and energy, are optimized for each line.
- The object inspected is exposed to a minimum ionizing-radiation dose.

### Brief description of the drawings

Embodiments of the invention are described in greater detail hereinafter relative to the attached schematic drawings.
- Figure 1: shows a diagrammatic view of the apparatus according to the invention.
- Figure 2: shows a slit collimator comprised in the apparatus according to the invention.
- Figure 3: shows a way of synchronizing the beam and the detector in the apparatus according to the invention.
- Figure 4: shows a flow chart of the method according to the invention.
- Figure 5: shows an area of preview and an area of interest in the method according to the invention.
- Figure 6: shows an adapted intensity and energy of the radiation in the method according to the invention.

### Description of preferred embodiments

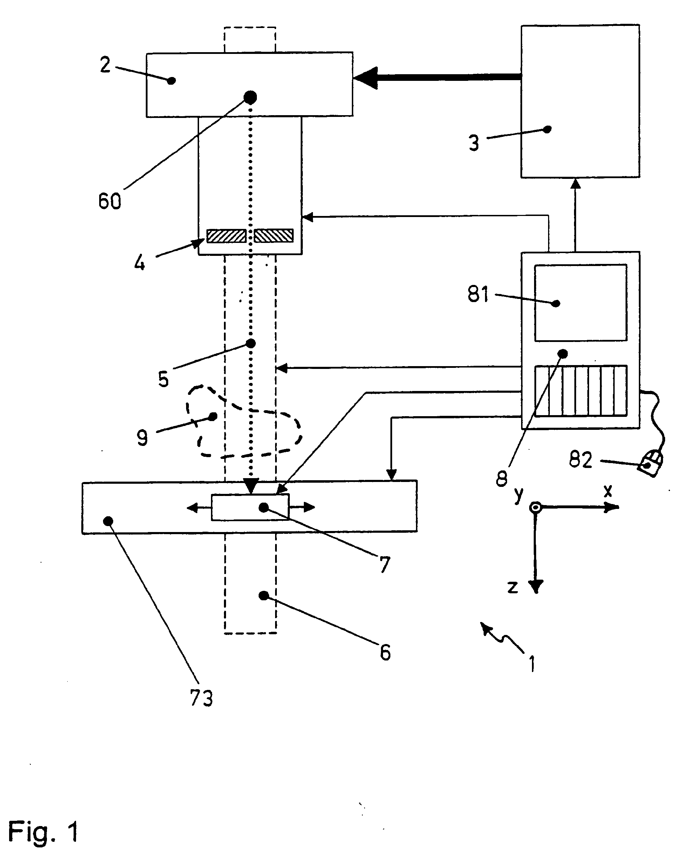

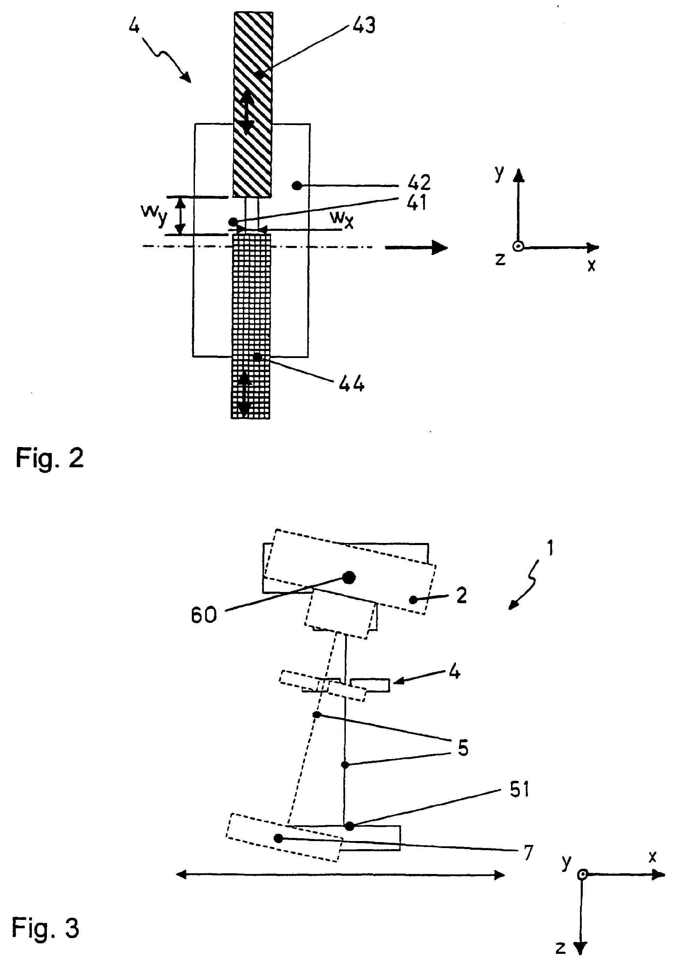

A preferred embodiment of the scanning digital radiography apparatus 1 according to the invention is diagrammatically shown in **Figure 1**. The apparatus 1 comprises an X-ray tube 2 energized by a high-voltage generator 3 whose excitation voltage and tube current can be dynamically remotely varied. The apparatus 1 further comprises a slit collimator 4 whose slit width, i.e., the width in a dimension y perpendicular to a direction x of scan, can be remotely and asymmetrically varied (cf. Fig. 2). Thus, radiation 5 behind the collimator 4 propagates in the form of a fan beam lying essentially in a fan-beam plane yz which is perpendicular to the scan direction x, the fan-beam angle being defined by the slit width. The tube 2 and the collimator 4 are mounted on a frame 6 with a drive such that they are rotatable about a pivot axis 60 lying preferably within the tube 2. A detector 7 with one or a few (e.g., up to ten) linear arrays of X-ray-sensitive picture elements (pixels) with digital output is arranged so as to detect X-rays emitted by the tube 2 and transmitted through an object 9 to be inspected. A transport mechanism 73 for the detector 7 linearly moves the detector in the scan direction x, or rotates the detector 7 with the tube 2 and the collimator 4 on the frame 6. A control unit 8 is provided for controlling the image acquisition, especially various scan movements and the parameters of the source 2. This is indicated in Fig. 1 by arrows pointing from the control unit 8 to various elements 3, 4, 6, 7, 73 of the apparatus 1. The control unit 8 also may serve as an interface with an operator and/or for processing the acquired images.

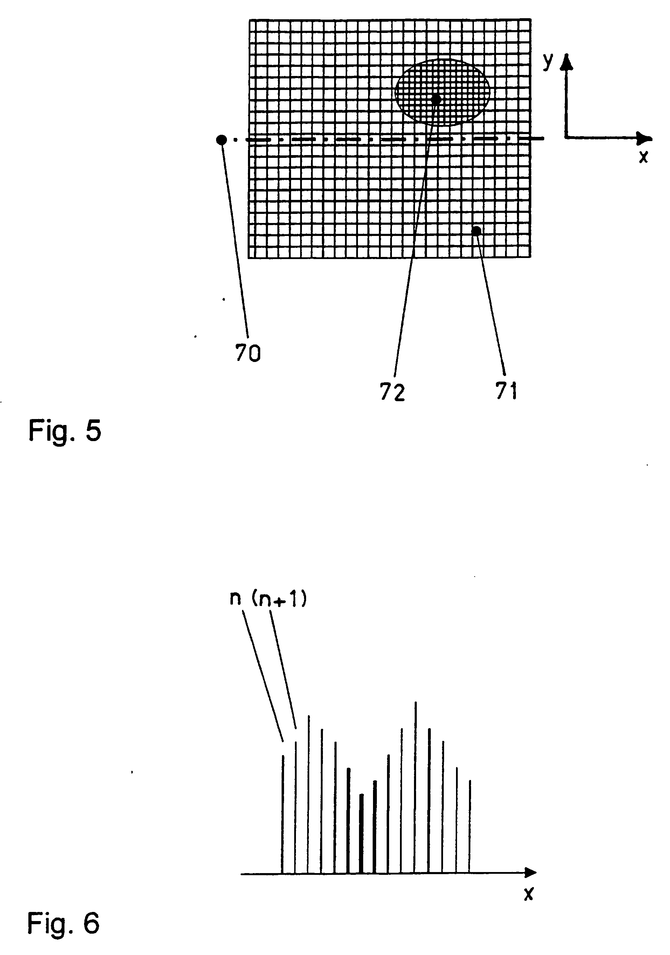

**Figure 2** shows a detail of the apparatus 1 according to the invention, namely, the remotely adaptive slit collimator 4. The collimator 4 comprises a first pair of shields 41, 42 and a second pair of shields 43, 44, both pairs of shields 41, 42; 43, 44 being essentially parallel. The first pair of shields 41, 42 defines the width (in the scan direction x) of the fan-shaped beam 5. The first slit width wₓ between the first pair of shields 41, 42 may be adjustable, but is preferably constant during a scan. The second pair of shields 43, 44 defines the fan angle of the fan-shaped beam 5, i.e., the length (in direction y perpendicular to the scan direction x) of the beam 5 on the detector array 7. Both shields 43, 44 of the second pair of shields are movable independently from each other in a direction y perpendicular to the scan direction x so as to vary the position y and/or the width wy of the second slit. Their movements during a scan are controlled by the control unit 8. Thus, by appropriately controlling the movements of the second pair of shields 43, 44 and by simultaneously scanning the object 9 in the scan direction x, an arbitrary (two-dimensional) area of interest 72 in the detector plane xy may be scanned (cf. Fig. 5). For most instances, the first slit width wₓ will be smaller than the second slit width w_{y}.

During a scan, the position of the detector 7 must be synchronized with the position of the fan-shaped beam 5. Preferably, also the angle of incidence 51 of the beam 5 on the detector 7 is synchronized to be near the optimum of 0° (perpendicular incidence) by tilting the detector 7, the tilt angle depending on the detector position. The synchronization of the X-ray beam 5 and the detector 7 can be achieved mechanically, such as shown in **Figure 3**, or by using multiple programmable positioning units, depending on the desired accuracy of synchronization. The velocity of the detector motion can be remotely adapted depending on the requirements of image quality and radiation dose. The synchronization and the velocity are preferably controlled by the control unit 8.

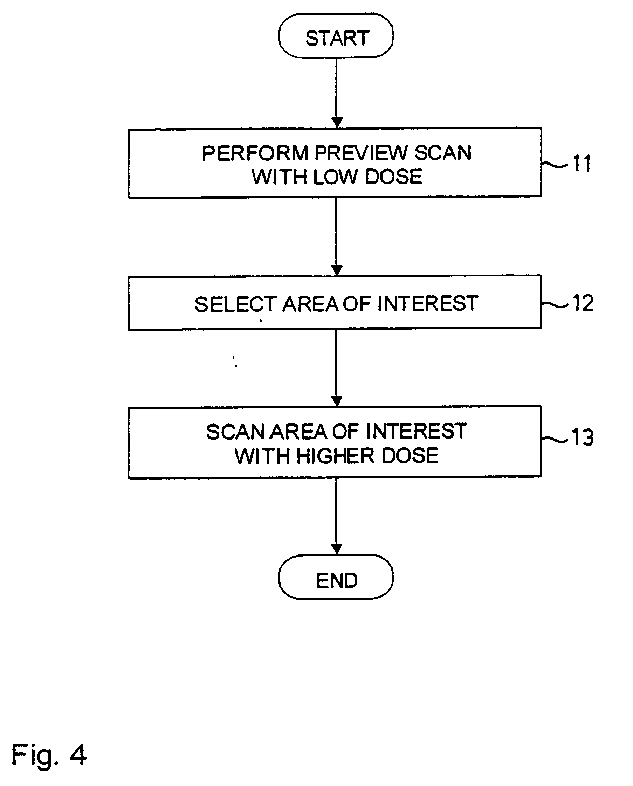

**Figure 4** illustrates the two-step line scanning approach of the method according to the invention. The method comprises the acquisition 11 of an extremely low-dose preview image(cf.
Fig. 5). Based upon the preview image, an area of interest 72 (cf. Fig. 5) is selected 12. Subsequently, a sound image of the area of interest 72 is acquired 13 by scanning the area of interest 72 with a higher dose. The dose used for the preview-image acquisition 11 is, e.g., 0.5-10 %, and preferably 2-4 % of the dose used for the sound-image acquisition 13.

During the image acquisition 13, the intensity and energy of the ionizing-radiation beam 5 (cf. Fig. 6), as well as the second slit width w_{y} of collimator 4(cf. Fig. 2) are controlled, and the movements of the detector 7 and the fan-shaped beam 5 are synchronized, preferably by the control unit 8. The control unit 8 may also be used for the visualization of the acquired images and the post processing of the acquired digital images according to the requirements of the application.

The area of interest 72 may be selected or defined by automatic means. For instance, the control unit 8 may comprise a processor capable of image recognition. The processor searches the preview image 71 (cf. Fig. 5) for certain patterns of interest, and if such a pattern of interest is found, automatically defines an area of interest 72 comprising the pattern of interest. Alternatively, an operator may select or define the area of interest 72. For this purpose, the control unit 8 may comprise an output device 81 for displaying the preview image, e.g., a monitor, and an input device 82 for defining the area of interest 72 with respect to the preview image 71, e.g., a computer mouse. Both alternatives may also be combined, e.g., by use of an adaptive processor capable of learning from the operator's inputs and taking over the definition of the area of interest after a certain learning phase.

An example of an area of preview 71 and a possible area of interest 72 is illustrated in **Figure 5**. The area of preview 71, scanned with a low radiation dose, is typically rectangular. The area of interest 72, scanned with intensities and energies necessary for a sound image, lies within the area of preview 71 and may have an arbitrary shape. In the simple example of Fig. 5, an elliptical area of interest 72 is drawn; however, its shape may be more complex. In particular, the area of interest 72 may consist of a plurality of partial areas which are not connected to each other, and it need not show any symmetry, e.g., to a center line 70 of the preview scan 71.

Finally, **Figure 6** shows a diagrammatic example of how the intensity and energy of the ionizing-radiation beam 5 may be varied and adapted during the scan 13 of the area of interest 72. A plurality of scan steps is performed at different positions, which are preferably equidistant in the scan direction x. For each scan step, the intensity and energy of the radiation is adapted to optimum conditions, e.g., with respect to the signal-to-noise ratio, determined by means of the preview scan 71. In Fig. 6, the intensity is indicated by the thickness and the energy is indicated by the length of a line at the corresponding position. In the example of Fig. 6, scan step n is performed with a higher intensity yet with a lower energy than step (n+1).

This invention is not limited to the preferred embodiments described above, to which variations and improvements may be made, without departing from the scope of protection of the present patent which is defined in the claims.

### List of reference signs

- 1: Apparatus
- 2: X-ray tube
- 3: High-voltage generator
- 4: Collimator
- 41, 42: First pair of shields
- 43, 44: Second pair of shields
- 5: Radiation beam
- 51: Angle of incidence
- 6: Frame
- 60: Pivot axis
- 7: Detector
- 70: Center line
- 71: Preview image
- 72: Area of interest
- 73: Transport mechanism
- 8: Control unit
- 81: Output device
- 82: Input device
- 9: Object
- 11: Acquisition of first image
- 12: Selection of area of interest
- 13: Acquisition of second image

- x, y, z: Cartesian coordinates
- wₓ, w_{y}: First and second slit width, respectively

## Claims

1. A method for imaging an object (9) with penetrating radiation, comprising the steps of:
generating a beam (5) of penetrating radiation, impinging said beam (5) on the object (9) and detecting radiation transmitted through the object (9), and
scanning the object (9) with said beam (5),
whereby
a first, preview image (71) is acquired (11) by scanning the object (9) or a part of it with a first radiation dose,
an area of interest (72) comprised in said preview image (71) is selected (12), a second image is acquired (13) by scanning said area of interest (72) with a second radiation dose which is higher than said first radiation dose,
during the acquisition (13) of said second image said beam's (5) energy spectrum and/or intensity is dynamically modulated, the method being **characterized in that**
for each scan step of said preview image (71), a local signal-to-noise ratio is calculated, compared to a predefined object-determined limit, and for each scan step of said second image, an optimum energy spectrum and/or intensity of said beam is determined according to the comparison between the local signal-to-noise ratio and the predefined object-determined limit, this optimum energy spectrum and/or intensity of said beam being dynamically modulated and applied in said second-image acquisition (13).

2. The method according to claim 1, wherein said beam's (5) energy spectrum and/or intensity is dynamically modulated by modulating an excitation voltage and/or an excitation current of a source (2) of said beam (5).

3. The method according to any of the preceding claims, wherein said beam (5) is generated so as to have a fan shape with a fan plane (yz) and two fan sides, and the object is scanned in a scan direction (x) perpendicular to said fan plane (yz).

4. The method according to claim 3, wherein both sides of said fan-shaped beam (5) are independently moved during the acquisition (13) of said second image in order to scan said area of interest (72), and the thickness (wₓ) of said fan-shaped beam (5) in the scan direction (x) is preferably constant.

5. The method according to any of the preceding claims, wherein said area of interest (72) is defined (12) automatically by applying an image recognition technique to said preview image (71).

6. The method according to any of the preceding claims, wherein said first radiation dose is 0.5-10 %, and preferably 2-4 % of said second radiation dose.

7. An apparatus (1) for imaging an object (9) with penetrating radiation, comprising
a source (2) for generating penetrating radiation,
beam-forming means (4) for forming a beam (5) from said radiation,
scanning means (6, 73) for scanning the object (9) with said beam (5),
detector means (7) for detecting radiation generated by said source (2) and transmitted through the object (9), and
control means (8) for controlling the imaging process,
**characterized in that**
said forming means (4) are suited for dynamically varying the geometry of said beam (5) during a scanning process, being controlled by said control means (8),
said control means (8) are adapted for
making said scanning means (6, 73) scan the object (9) or a part of it with a first radiation dose so as to acquire a first, preview image (71),
selecting an area of interest (72) comprised in said preview image (71), and
making said scanning means (6, 73) scan said area of interest (72) with a second radiation dose which is higher than said first radiation dose so as to acquire a second image,
said control means (8) being further adapted for modulating the energy spectrum and/or intensity of the radiation emitted by said source,
calculating a local signal-to-noise ratio for each scan step of said preview image (71) and comparing it to a predefined object-determined limit, and
determining an optimum energy spectrum and/or intensity of the radiation emitted by said source (2) for each scan step of said second image according to the comparison between the local signal-to-noise ratio and the predefined object-determined limit and
applying this optimum energy spectrum and/or intensity of the radiation emitted by said source (2) in said second image acquisition.

8. The apparatus (1) according to claim 7, wherein said beam-forming means (4) comprise a first pair of shields (41, 42) defining a first slit and a second pair of shields (43, 44) defining a second slit arranged essentially perpendicular to said first slit, both pairs of shields (41, 42; 43, 44) being essentially parallel, and both shields (43, 44) of said second pair of shields being movable independently from each other so as to vary the position (y) and/or the width (w_{y}) of said second slit.

9. The apparatus (1) according to claim 8, wherein said scanning means (6, 73) are adapted for scanning in a scan direction (x), said first slit being perpendicular to said scan direction (x) and said second slit being parallel to said scan direction (x).

10. The apparatus (1) according to claim any of claims 7-9, wherein said control means (8) are adapted for modulating the energy spectrum and/or intensity of the radiation emitted by said source by modulating an excitation voltage and/or an excitation current of said source.

11. The apparatus (1) according to any of claims 7-10, wherein said control means (8) comprise an output device (81) for displaying said preview image (71), e.g., a monitor, and an input device (82) for defining said area of interest (72) with respect to said preview image (71), e.g., a computer mouse.

12. The apparatus (1) according to any of claims 7-11, wherein said source (2) is an X-ray tube.

13. The apparatus (1) according to any of claims 7-12, wherein said scanning means (6, 73) comprise means (6) for linearly moving or rotating said source (2) and said beam-forming means (4) with respect to the object (9), and/or means (73) for linearly moving or rotating said detector means (7) with respect to the object (9).

14. The apparatus (1) according to any of claims 7-13, wherein said detector means (7) comprise at least one linear array of pixels.

## Patentansprüche

1. Verfahren zum Abbilden eines Objekts (9) mit einer durchdringenden Strahlung, umfassend die folgenden Schritte:
Erzeugen eines Strahls (5) einer durchdringenden Strahlung, Auftreffen lassen des Strahls (5) auf das Objekt (9) und Feststellen der Strahlung, die durch das Objekt (9) übertragen wurde, und
Abtasten des Objekts (9) mit dem Strahl (5),
wobei
durch Abtasten des Objekts (9) oder eines Teils davon mit einer ersten Strahlungsdosis ein erstes Vorschaubild (71) erlangt (11) wird,
ein Bereich von Interesse (72), der in diesem Vorschaubild (71) beinhaltet ist, gewählt wird (12), durch Abtasten des Bereichs von Interesse (72) mit einer Strahlungsdosis, die höher als die erste Strahlungsdosis ist, ein zweites Bild erlangt wird (13),
während der Erlangung (13) des zweiten Bilds das Energiespektrum und/oder die Intensität des Strahls (5) dynamisch moduliert wird, wobei das Verfahren **dadurch gekennzeichnet ist, dass**
für jeden Abtastschritt des Vorschaubilds (71) ein lokales Signal-Rausch-Verhältnis berechnet wird, mit einer vordefinierten objektbestimmten Grenze verglichen wird, und für jeden Abtastschritt des zweiten Bilds ein optimales Energiespektrum und/oder eine optimale Intensität des Strahls gemäß dem lokalen Signal-Rausch-Verhältnis und der vordefinierten objektbestimmten Grenze bestimmt wird, wobei dieses optimale Energiespektrum und/oder diese optimale Intensität des Strahls dynamisch moduliert und bei der Erlangung (13) des zweiten Bilds angewendet werden.

2. Verfahren nach Anspruch 1, wobei das Energiespektrum und/oder die Intensität des Strahls (5) dynamisch moduliert werden, indem eine Anregungsspannung und/oder ein Anregungsstrom einer Quelle (2) des Strahls (5) moduliert werden.

3. Verfahren nach einem der vorhergehenden Ansprüche, wobei der Strahl (5) so erzeugt wird, dass er eine Fächerform mit einer Fächerebene (yz) und zwei Fächerseiten aufweist, und das Objekt in einer Abtastrichtung (x) senkrecht zur Fächerebene (yz) abgetastet wird.

4. Verfahren nach Anspruch 3, wobei beide Seiten des fächerförmigen Strahls (5) während der Erlangung (13) des zweiten Bilds unabhängig bewegt werden, um den Bereich von Interesse (72) abzutasten, und die Dicke (wₓ) des fächerförmigen Strahls (5) in der Abtastrichtung (x) vorzugsweise konstant ist.

5. Verfahren nach einem der vorhergehenden Ansprüche, wobei der Bereich von Interesse (72) automatisch definiert wird (12), indem eine Bilderkennungstechnik auf das Vorschaubild (71) angewendet wird.

6. Verfahren nach einem der vorhergehenden Ansprüche, wobei die erste Strahlungsdosis 0,5 bis 10 % und vorzugsweise 2 bis 4 % der zweiten Strahlungsdosis beträgt.

7. Vorrichtung (1) zum Abbilden eines Objekts (9) mit einer durchdringenden Strahlung, umfassend
eine Quelle (2) zum Erzeugen der durchdringenden Strahlung,
ein Strahlbildungsmittel (4) zum Bilden eines Strahls (5) aus der Strahlung,
ein Abtastmittel (6, 73) zum Abtasten des Objekts (9) mit dem Strahl (5),
ein Detektormittel (7) zum Feststellen der Strahlung, die durch die Quelle (2) erzeugt wurde und durch das Objekt (9) übertragen wurde, und
ein Steuermittel (8) zum Steuern des Abbildungsprozesses,
**dadurch gekennzeichnet, dass**
das Bildungsmittel (4) dazu geeignet ist, die Geometrie des Strahls (5) während eines Abtastprozesses unter Steuerung durch das Steuermittel (8) dynamisch zu verändern,
wobei das Steuermittel (8) dazu geeignet ist,
das Abtastmittel (6, 73) den Gegenstand (9) oder einen Teil davon mit einer ersten Strahlungsdosis abtasten zu lassen, um ein erstes Vorschaubild (71) zu erlangen,
einen Bereich von Interesse (72) zu wählen, der im Vorschaubild (71) beinhaltet ist, und
das Abtastmittel (6, 73) den Bereich von Interesse (72) mit einer zweiten Strahlungsdosis, die höher als die erste Strahlungsdosis ist, abtasten zu lassen, um ein zweites Bild zu erlangen,
wobei das Steuermittel (8) ferner dazu geeignet ist, das Energiespektrum und/oder die Intensität der durch die Quelle abgegebenen Strahlung zu modulieren,
für jeden Abtastschritt des Vorschaubilds (71) ein lokales Signal-Rausch-Verhältnis zu berechnen und dieses mit einer vordefinierten objektbestimmten Grenze zu vergleichen, und
für jeden Abtastschritt des zweiten Bilds ein optimales Energiespektrum und/oder eine optimale Intensität der durch die Quelle (2) abgegebenen Strahlung gemäß dem Vergleich zwischen dem lokalen Signal-Rausch-Verhältnis und der vordefinierten objektbestimmten Grenze zu bestimmen, und
dieses optimale Energiespektrum und/oder diese optimale Intensität der durch die Quelle (2) abgegebenen Strahlung bei der zweiten Bilderlangung anzuwenden.

8. Vorrichtung (1) nach Anspruch 7, wobei das Strahlbildungsmittel (4) ein erstes Paar von Abschirmungen (41, 42) umfasst, die einen ersten Schlitz definieren, und ein zweites Paar von Abschirmungen (43, 44) umfasst, die einen zweiten Schlitz definieren, der im Wesentlichen senkrecht zum ersten Schlitz angeordnet ist, wobei beide Paare von Abschirmungen (41, 42; 43, 44) im Wesentlichen parallel sind, und wobei beide Abschirmungen (43, 44) des zweiten Paars von Abschirmungen unabhängig voneinander beweglich sind, um die Position (y) und/oder die Breite (w_{y}) des zweiten Schlitzes zu verändern.

9. Vorrichtung (1) nach Anspruch 8, wobei das Abtastmittel (6, 73) dazu geeignet ist, in einer Abtastrichtung (x) abzutasten, wobei der erste Schlitz senkrecht zur Abtastrichtung (x) und der zweite Schlitz parallel zur Abtastrichtung (x) verläuft.

10. Vorrichtung (1) nach einem der Ansprüche 7 bis 9, wobei das Steuermittel (8) dazu geeignet ist, das Energiespektrum und/oder die Intensität der von der Quelle abgegebenen Strahlung durch Modulieren einer Anregungsspannung und/oder eines Anregungsstroms der Quelle zu modulieren.

11. Vorrichtung (1) nach einem der Ansprüche 7 bis 10, wobei das Steuermittel (8) eine Ausgabevorrichtung (81) zum Darstellen des Vorschaubilds (71), z.B. einen Bildschirm, und eine Eingabevorrichtung (82) zum Definieren des Bereichs von Interesse (72) in Bezug auf das Vorschaubild (71), z.B. eine Computermaus, umfasst.

12. Vorrichtung (1) nach einem der Ansprüche 7 bis 11, wobei die Quelle (2) eine Röntgenröhre ist.

13. Vorrichtung (1) nach einem der Ansprüche 7 bis 12, wobei das Abtastmittel (6, 73) ein Mittel (6) zum linearen Bewegen oder Drehen der Quelle (2) und des Strahlformungsmittels (4) in Bezug auf das Objekt (9) und/oder ein Mittel (73) zum linearen Bewegen oder Drehen des Detektormittels (7) in Bezug auf das Objekt (9) umfasst.

14. Vorrichtung (1) nach einem der Ansprüche 7 bis 13, wobei das Detektormittel (7) zumindest eine lineare Anordnung von Pixeln umfasst.

## Revendications

1. Méthode pour imager un objet (9) avec des rayonnements pénétrants, comprenant les étapes suivantes :
génération d'un faisceau (5) de rayonnements pénétrants, convergence dudit faisceau (5) avec l'objet (9) et détection des rayonnements transmis à travers l'objet (9), et
balayage de l'objet (9) avec ledit faisceau (5),
dans laquelle
une première image de prévisualisation (71) est acquise (11) en balayant l'objet (9) ou un partie de celui-ci avec une première dose de rayonnements,
une zone d'intérêt (72) comprise dans ladite image de prévisualisation (71) est sélectionnée (12), et une deuxième image est acquise (13) en balayant ladite zone d'intérêt (72) avec une deuxième dose de rayonnements supérieure à ladite première dose de rayonnements,
pendant l'acquisition (13) de ladite deuxième image, le spectre d'énergie et/ou l'intensité dudit faisceau (5) sont modulés dynamiquement, la méthode étant **caractérisée en ce que**
pour chaque étape de balayage de ladite image de prévisualisation (71), un rapport local signal/bruit est calculé, comparé à une limite déterminée / à un objet prédéfini, et pour chaque étape de balayage de ladite deuxième image, un spectre d'énergie optimal et/ou une intensité optimale sont définis pour ledit faisceau, en fonction de la comparaison entre le rapport local signal/bruit et un objet prédéfini / une limite déterminée, ce spectre d'énergie optimal et/ou cette intensité optimale dudit faisceau étant modulés dynamiquement et appliqués dans ladite acquisition (13) de deuxième image.

2. Méthode selon la revendication 1, dans laquelle ledit spectre d'énergie et/ou ladite intensité du faisceau (5) sont modulés dynamiquement en modulant une tension d'excitation et/ou un courant d'excitation venant d'une source (2) dudit faisceau (5).

3. Méthode selon l'une quelconque des revendications précédentes, dans laquelle ledit faisceau (5) est généré de façon à avoir une forme d'éventail, avec un plan d'éventail (yz) et deux côtés d'éventail, et l'objet est balayé dans un sens de balayage (x) qui est perpendiculaire audit plan d'éventail (yz).

4. Méthode selon la revendication 3, **caractérisée en ce que** les deux côtés du faisceau en forme d'éventail (5) sont déplacés indépendamment l'un de l'autre pendant l'acquisition (13) de ladite deuxième image, afin de balayer ladite zone d'intérêt (72), est **en ce que** l'épaisseur (wₓ) dudit faisceau (5) en forme d'éventail dans le sens de balayage (x) est de préférence constante.

5. Méthode selon lune quelconque des revendications précédentes, dans laquelle ladite zone d'intérêt (72) est définie (12) automatiquement, en appliquant une technique de reconnaissance d'image à ladite image de prévisualisation (71).

6. Méthode selon l'une quelconque des revendications précédentes, dans laquelle ladite première dose de rayonnements mesure entre 0,5 et 10%, et de préférence entre 2 et 4% % de ladite deuxième dose de rayonnements.

7. Appareil (1) pour imager un objet (9) avec des rayonnements pénétrants, comprenant les éléments suivants :
une source (2) pour la génération de rayonnements pénétrants,
des moyens de formation de faisceaux (4) pour la formation d'un faisceau (5) à partir desdits rayonnements,
des moyens de balayage (6, 73) pour le balayage de l'objet (9) avec ledit faisceau (5),
des moyens de détection (7) pour la détection des rayonnements générés par ladite source (2) et transmis à travers l'objet (9), et
des moyens de contrôle (8) pour le contrôle du processus d'imagerie,
**caractérisé en ce que**
lesdits moyens de formation (4) étant adaptés pour faire varier dynamiquement la géométrie dudit faisceau (5) pendant le processus de balayage, sous le contrôle desdits moyens de contrôle (8),
lesdits moyens de contrôle (8) étant adaptés pour
veiller à ce que lesdits moyens de balayage (6, 73) balaient l'objet (9) ou une partie de celui-ci, avec une première dose de rayonnements, de façon à acquérir une première image de prévisualisation (71),
sélectionner une zone d'intérêt (72) comprise dans ladite image de prévisualisation (71), et
veiller à ce que lesdits moyens de balayage (6, 73) balaient ladite zone d'intérêt (72) avec une deuxième dose de rayonnements supérieure à la première dose de rayonnements, de façon à acquérir une deuxième image,
lesdits moyens de contrôle (8) étant en outre adaptés pour moduler le spectre d'énergie et/ou l'intensité des rayonnements émis par ladite source,
calculer un rapport local signal/bruit pour chaque étape de balayage de ladite image de prévisualisation (71), et le comparer à un objet prédéfini / à une limite déterminée, et
déterminer un spectre d'énergie optimal et/ou une intensité optimale des rayonnements émis par ladite source (2), pour chaque étape de balayage de ladite deuxième image, en fonction de la comparaison entre le rapport local signal/bruit et l'objet prédéfini / la limite déterminée, et
appliquer ce spectre d'énergie optimal et/ou cette intensité optimale de rayonnements émis par ladite source (2) à ladite deuxième image d'acquisition.

8. Appareil (1) selon la revendication 7, dans lequel lesdits moyens de formation de faisceaux (4) comprennent une première paire d'écrans (41, 42) définissant une première fente, ainsi qu'une deuxième paire d'écrans (43, 44) définissant une deuxième fente disposée essentiellement perpendiculairement à ladite première fente, les deux paires d'écrans (41, 42; 43, 44) étant essentiellement parallèles, et les deux écrans (43, 44) de ladite deuxième paire d'écrans étant mobiles indépendamment l'un de l'autre, de façon à faire varier la position (y) et/ou la largeur (w_{y}) de ladite deuxième fente.

9. Appareil (1) selon la revendication 8, dans lequel lesdits moyens de balayage (6, 73) sont adaptés pour balayer dans un sens de balayage (x), ladite première fente étant perpendiculaire audit sens de balayage (x), et ladite deuxième fente étant parallèle audit sens de balayage (x).

10. Appareil (1) selon l'une quelconque des revendications 7 à 9, dans lequel lesdits moyens de contrôle (8) sont adaptés pour moduler le spectre d'énergie et/ou l'intensité des rayonnements émis par ladite source, en modulant une tension d'excitation et/ou un courant d'excitation de ladite source.

11. Appareil (1) selon l'une quelconque des revendications 7 à 10, dans lequel lesdits moyens de contrôle (8) comprennent un dispositif de sortie (81) pour afficher ladite image de prévisualisation (71), par exemple un moniteur, ainsi qu'un dispositif d'entrée (82) pour définir ladite zone d'intérêt (72) par rapport à ladite image de prévisualisation (71), par exemple une souris d'ordinateur.

12. Appareil (1) selon l'une quelconque des revendications 7 à 11, dans lequel ladite source (2) est un tube à rayons X.

13. Appareil (1) selon l'une quelconque des revendications 7 à 12, dans lequel lesdits moyens de balayage (6, 73) comprennent des moyens (6) pour déplacer de façon linéaire ou faire tourner ladite source (2) et lesdits moyens de formation de faisceaux (4) par rapport à l'objet (9), et/ou des moyens (73) pour déplacer de façon linéaire ou faire tourner lesdits moyens de détection (7) par rapport à l'objet (9).

14. Appareil (1) selon l'une quelconque des revendications 7 à 13, dans lequel lesdits moyens de détection (7) comprennent au moins une barrette de pixels.
